# EUROPEAN PATENT APPLICATION

(11) **EP 2 172 217 A1**
(43) Date of publication of application: **07.04.2010**
(21) Application number: 08765268.1
(22) Date of filing: 06.06.2008
(51) Int. Cl.: A61K 39/395, A61K 45/00, A61P 1/04, A61P 1/16, A61P 1/18, A61P 9/00, A61P 11/00, A61P 13/12, A61P 25/16

(54) **FIBROSIS INHIBITOR**

(30) Priority: 07.06.2007 JP 2007151164
(71) Applicant: Stelic Institute Of Regenerative Medicine, Stelic Institute & Co., Tokyo 106-0044 (JP)
(72) Inventor: YONEYAMA, Hiroyuki, Tokyo 106-0044 (JP); KAI, Yoshiro, Kashihara-shi Nara 634-0004 (JP); TAGASHIRA, Hideki, Tokyo 106-0044 (JP); KOYAMA, Jun, Tokyo 106-0044 (JP)
(74) Representative: Grünecker, Kinkeldey, Stockmair & Schwanhäusser Anwaltssozietät
(86) International application number: PCT/JP2008/060455
(87) International publication number: WO 2008/149980

(57) **Abstract**

An objective of the present invention is to provide methods for treating or preventing fibrotic diseases, which are based on the novel finding that fibrosis is suppressed by administering ER-TR7 to a subj ect.

SEQUENCE LISTING

<110> STELIC INSTITUTE OF REGENERATIVE MEDICINE
STELIC INSTITUTE & CO.

<120> Fibrosis inhibitor

<130> EP68330HVpau

<140> not yet assigned
<141> herewith

<150> PCT/JP2008/060455
<151> 2007-06-06

<150> JP 2007-151164
<151> 2007-06-07

<160> 12

<170> PatentIn version 3.3

<210> 1
<211> 25
<212> DNA
<213> Artificial

<220>
<223> An artificially synthesized primer sequence

<400> 1
catccgtaaa gacctctatg ccaac 25

<210> 2
<211> 19
<212> DNA
<213> Artificial

<220>
<223> An artificially synthesized primer sequence

<400> 2
atggagccac cgatccaca 19

<210> 3
<211> 20
<212> DNA
<213> Artificial

<220>
<223> An artificially synthesized primer sequence

<400> 3
aaatggtgaa ggtcggtgtg 20

<210> 4
<211> 18
<212> DNA
<213> Artificial

<220>
<223> An artificially synthesized primer sequence

<400> 4
tgaaggggtc gttgatgg 18

<210>  5
<211>  19
<212>  DNA
<213>  Artificial

<220>
<223>  An artificially synthesized primer sequence

<400>  5
ttccaggctt tgggcatca                                                    19


<210>  6
<211>  24
<212>  DNA
<213>  Artificial

<220>
<223>  An artificially synthesized primer sequence

<400>  6
atgttcagca tgttcagcag tgtg                                              24


<210>  7
<211>  23
<212>  DNA
<213>  Artificial

<220>
<223>  An artificially synthesized primer sequence

<400>  7
caggagggag aacagaaact cca                                               23


<210>  8
<211>  19
<212>  DNA
<213>  Artificial

<220>
<223>  An artificially synthesized primer sequence

<400>  8
cctggttggc tgcttgctt                                                    19


<210>  9
<211>  19
<212>  DNA
<213>  Artificial

<220>
<223>  An artificially synthesized primer sequence

<400>  9
acccgatggc aacaatgga                                                    19


<210>  10
<211>  20
<212>  DNA
<213>  Artificial

<220>

<223>  An artificially synthesized primer sequence

<400>  10
accagcaggg ccttgttcac                                                    20


<210>  11
<211>  21
<212>  DNA
<213>  Artificial

<220>
<223>  An artificially synthesized primer sequence

<400>  11
gagcatccga cactgctgac a                                                  21


<210>  12
<211>  24
<212>  DNA
<213>  Artificial

<220>
<223>  An artificially synthesized primer sequence

<400>  12
agcacagcct gaatagccac atac                                               24

## Description

### Technical Field

The present invention relates to agents for treating or preventing fibrotic diseases, and uses thereof.

### Background Art

Fibrosis is an uncontrolled wound healing response. An injured tissue is repaired by replacing damaged cells with cells of the same type, but occasionally, parenchymal tissues are replaced by connective tissues. In such cases, fibroblasts become activated and differentiate into myoblasts, leading to synthesis, deposition, formulation, and remodeling of the extracellular matrix. This results in fibrosis. Chronic fibrotic disease is a general term for the terminal stage of chronic inflammatory conditions that can develop in various organs, and is accompanied by excessive tissue fibrosis. Chronic fibrotic disease includes, for example, cirrhosis of the liver and sclerosis of the kidney. Furthermore, these fibrotic diseases progress into organ failure or cancer (Non-patent Document 1).

A colonic disease known to involve fibrosis is Crohn's disease, which has been designated as a specified disease by the Ministry of Health, Labor, and Welfare of Japan. Crohn's disease has been defmed as "a disease of uncertain etiology mostly seen in young subjects, which involves granulomatous inflammatory focus with fibrosis and ulcers, can affect any part of the gastrointestinal tract from the oral cavity to anus, and may also affect areas other than the gastrointestinal tract (specifically skin)". Most lesion areas have longitudinal ulcers, cobblestone appearances, or aphtha in the small or large intestine, or both. Crohn's disease is categorized into disease types such as longitudinal ulcer type and cobblestone appearance type, or small intestine type, small/large intestine type, and large intestine type according to the location of stenosis. Common symptoms include abdominal pain, diarrhea, weight loss, fever, and anal lesions. Occasionally, the disease may show symptoms similar to those of appendicitis, ileus, intestinal perforation, or massive bleeding. Alternatively, the disease may develop with anal lesions or fever, without abdominal symptoms. Extragastrointestinal complications include anemia, hypoproteinemia, ankylosing spondylitis, aphthous stomatitis, erythema nodosum, pyoderma gangrenosum, iritis, and growth disorders.

In Japan, the former Ministry of Health and Welfare set up a study group in 1975, defined diagnostic criteria for Crohn's disease, and implemented a nationwide survey. The number of patients has been increasing every year since the start of the survey. The number of Medical Care Certificates issued was 128 in 1976, but was increased to 24,396 in 2007. The most common age at onset ranges from 20 to 24 in men, and 15 to 19 in women. The male-to-female ratio is about 2:1. Thus, Crohn's disease is more common in men.

As for other chronic fibrotic diseases, carriers of hepatitis virus, which is a major cause of progression to cirrhosis, are estimated to be 1,200,000 to 1,400,000 for type B and 1,000,000 to 2,000,000 for type C, according to blue-ribbon panel report on hepatitis countermeasures presented in 2001. Furthermore, the number of patients who undergo dialysis due to chronic renal failure caused by kidney fibrosis was approximately 250,000 according to the fundamental survey on chronically-dialyzed patients conducted by the Japan Society for Dialysis Therapy in 2004. The number of dialysis patients was 35,000, up 3.2% from the previous year, and increasing each year.

At present, there is no radical therapeutic method for completely curing fibrotic diseases. The reason is that the cause and pathogenesis is not understood. Presently, the purpose of treatment is to improve patients' quality of life (QOL) through maintaining relief from symptoms by controlling the activeness of the disease. The ultimate form of treatment is dialysis or organ transplantation. However, these therapeutic methods significantly impair patients' QOL, and many cases show complications and recurrence of fibrosis in a transplanted organ. Pathologically, fibrotic diseases are characterized by overaccumulation of extracellular matrix such as collagen. The overactivation of fibroblasts is recently assumed to be the major cause of the overaccumulation. Thus, fibroblasts are drawing attention as a therapeutic target.

Currently, as candidate therapeutic agents for fibrotic diseases, hope is placed on adrenal cortical steroids, colchicine, IL-10, and the like, which suppress chronic inflammation; TGF-β inhibitors, HGF (suppressing the activity of TGF-β), endothelin inhibitors, angiotensin inhibitors, and such, which activate fibroblasts; matrix metalloproteinase (MMP) which degrades collagen, etc. However, none has lead to an established therapeutic method.

ER-TR7 is a rat monoclonal antibody against non-lymphocytic cells of mouse thymus, which was produced by Van Vliet *et al.* in 1984 (Non-patent Document 2). ER-TR7 has been reported as an antibody that recognizes reticular fibroblasts (Non-patent Document 3), and is commercially available from several suppliers as a connective tissue marker for various organs. However, the antigen is yet unidentified. Recently, it was reported that, when contacted with lymphocytes, lymph node fibroblastic reticular cells contribute to the immunological function by secreting ER-TR7 antigen as an extracellular matrix to make a reticular meshwork (Non-patent Document 4).
[Non-patent Document 1] Wynn TA., J. Clin. Invest., (2007) 117: 524-529
[Non-patent Document 2] Van Vliet E. et al., Eur. J. Immunol., (1984) 14: 524-529
[Non-patent Document 3] Van Vliet E. et al., J. Histochem. Cytochem., (1986) 34: 883-890
[Non-patent Document 4] Katakai T. et al., J. Exp. Med., (2004) 200: 783-795

### Disclosure of the Invention

### [Problems to be Solved by the Invention]

An objective of the present invention is to provide agents that can suppress tissue fibrosis, which are useful in treating or preventing fibrotic diseases.

### [Means for Solving the Problems]

The present inventors suspected that ER-TR7, a monoclonal antibody that has been used as a fibrosis marker, may neutralize fibrosis-enhancing substances and thus have the effect of suppressing fibrosis. The present inventors carried out research based on this assumption and as a result, discovered that ER-TR7 can suppress fibrosis. Thus, the present invention was completed. ER-TR7 and functionally equivalent substances are effective fibrosis-suppressing agents treating or preventing fibrosis.

The present invention relates to agents for suppressing fibrosis and therapeutic agents for fibrotic diseases comprising the agents as an active ingredient, more specifically, provides the following:
[1] a fibrosis-suppressing agent comprising ER-TR7 or a functionally equivalent substance as an active ingredient;
[2] the agent of [1], wherein the substance has an ER-TR7 antigen-targeting activity;
[3] the agent of [1], wherein the substance has an activity of enhancing the degradation of an ER-TR7 antigen;
[4] the agent of [1], wherein the substance has an activity of inhibiting the synthesis of an ER-TR7 antigen;
[5] the agent of [1], wherein the substance has an activity of neutralizing the activity of an ER-TR7 antigen;
[6] the agent of [1], wherein the substance has an activity of inhibiting the modification of an ER-TR7 antigen;
[7] the agent of [1], wherein the substance has an activity of enhancing the demodification of an ER-TR7 antigen;
[8] the agent of any one of [1] to [7], wherein the production or accumulation of an ER-TR7 antigen in each organ is inhibited;
[9] the agent of any one of [1] to [8], which is used for treating or preventing a fibrotic disease (the fibrotic diseases are not particularly limited, and include, for example, various diseases caused by fibrosis of body tissues such as organs and skin, which include cirrhosis, renal sclerosis, pulmonary fibrosis, scleroderma, cardiomyopathy, myelofibrosis, and chronic peritonitis);
[10] a method for producing a fibrosis-suppressing agent comprising as an active ingredient an antibody, which comprises the step of preparing the antibody using an ER-TR7 antigen;
[11] a method for treating or preventing a fibrotic disease, which comprises the step of administering the agent of any one of [1] to [9] to a subject; and
[12] the use of ER-TR7 or a functionally equivalent substance in producing a fibrosis-suppressing agent.

### [Effects of the Invention]

The present invention revealed that ER-TR7 antigen is involved in the onset and promotion of fibrosis. The present invention also demonstrated that the onset of fibrosis was suppressed by neutralizing ER-TR7 antigen. These findings are applicable to provide unprecedented novel-concept fibrosis-suppressing agents. Since fibrosis occurs in various organs and can lead to severe diseases, the agents have great medical and industrial significances.

### Brief Description of the Drawings

Fig. 1 shows photographs depicting the therapeutic effect of ER-TR7 in cirrhosis model mice. (1) immunostaining image for type 4 collagen; (2) Masson staining image.
Fig. 2 shows the suppression of fibrosis marker gene expression as a result of ER-TR7 administration into cirrhosis model mice. (1) to (3) show alterations in the expression levels of α-SMA, TNFα, and type 1 collagen, respectively, determined by qPCR.
Fig. 3 shows the therapeutic effect of ER-TR7 in ulcerative colitis model mice. This diagram shows the result of intestinal length measurement in a control group, and ulcerative colitis model groups (untreated and ER-TR7-treated).
Fig. 4 shows the suppression of fibrosis marker gene expression as a result of ER-TR7 administration in ulcerative colitis model mice. (1) to (3) show alterations in the expression levels of α-SMA, TNFα, and type 1 collagen, respectively, determined by qPCR.
Fig. 5 shows photographs depicting the expression of ER-TR7 antigen in normal human lung fibroblasts in the presence or absence of lipopolysaccharide (LPS). The photographs show staining images for ER-TR7 in normal human lung fibroblasts.
Fig. 6 shows photographs depicting electrophoresis images of normal human lung fibroblast extracts. The photographs show images of Coomassie brilliant blue staining and Western blot.

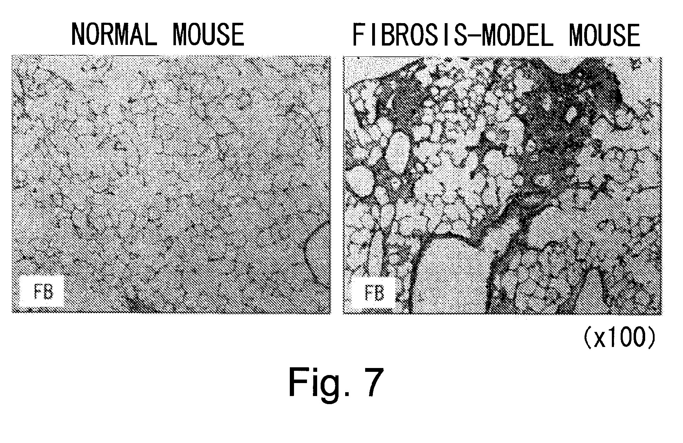

Fig. 7 shows photographs depicting the accumulation of ER-TR7-positive cells in lesions of a fibrosis model. Namely, it shows the result for lung tissue of pulmonary emphysema model mice. Antibody binding was detected by visualizing as a brown signal.

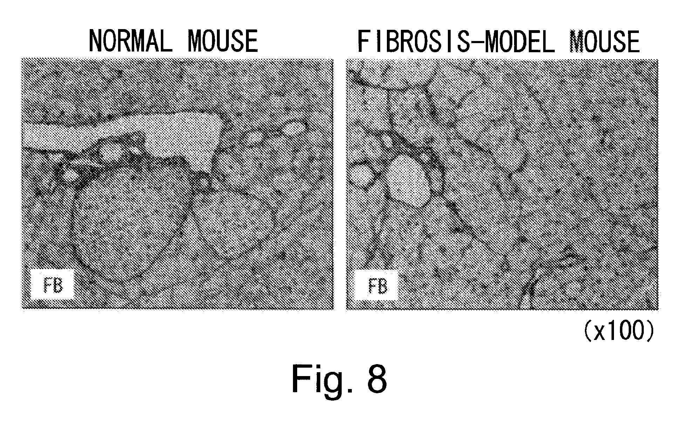

Fig. 8 shows photographs depicting the accumulation of ER-TR7-positive cells in lesions of a fibrosis model. Namely, it shows the result for pancreatic tissue of type 2 diabetes model mice. Antibody binding was detected by visualizing it as a brown signal.

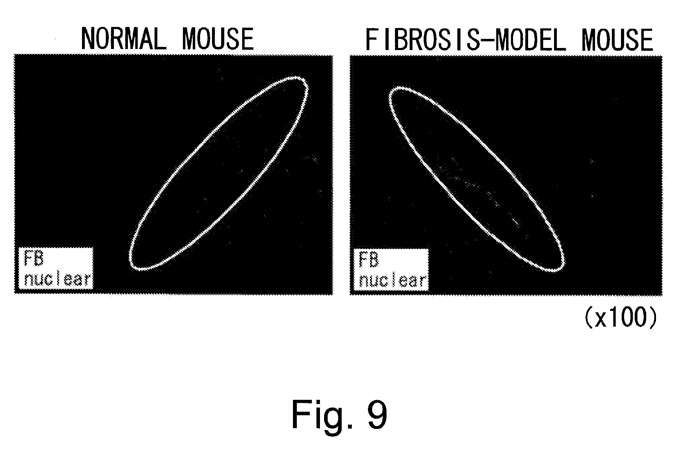

Fig. 9 shows photographs depicting the accumulation of ER-TR7-positive cells in lesions of a fibrosis model. Namely, it shows the result for brain tissue of Parkinson's disease model mice. Antibody binding was detected.

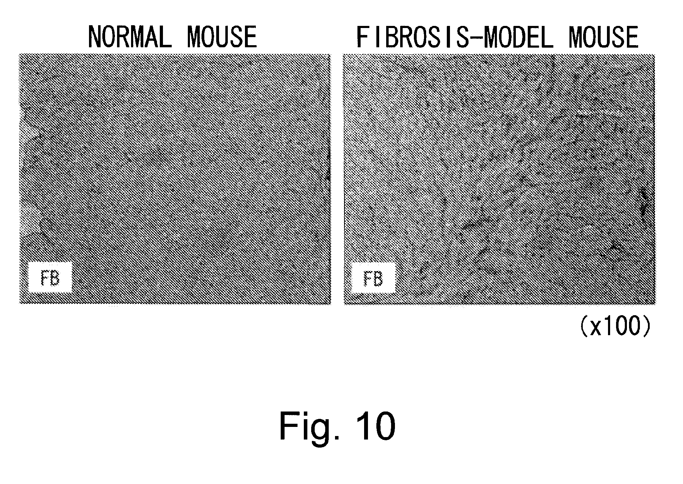

Fig. 10 shows photographs depicting the accumulation of ER-TR7-positive cells in lesions of a fibrosis model. Namely, it shows the result for cardiac tissue of cardiomyopathy model mice. Antibody binding was detected by visualizing it as a brown signal.

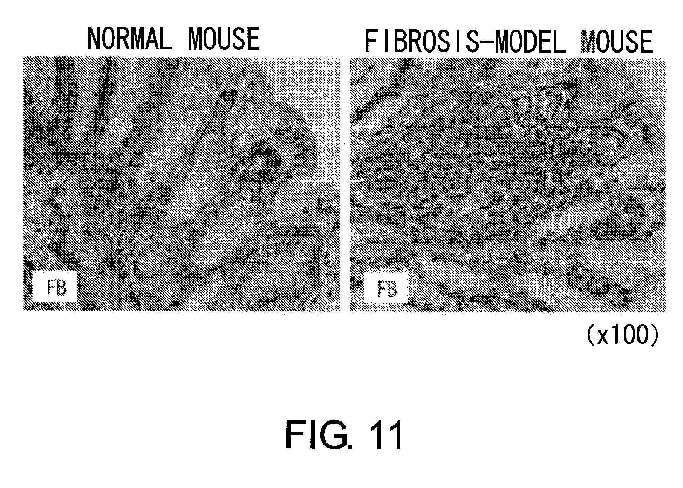

Fig. 11 shows photographs depicting the accumulation of ER-TR7-positive cells in lesions of a fibrosis model. Namely, it shows the result for colonic tissue of ulcerative colitis model mice. Antibody binding was detected by visualizing it as a brown signal.

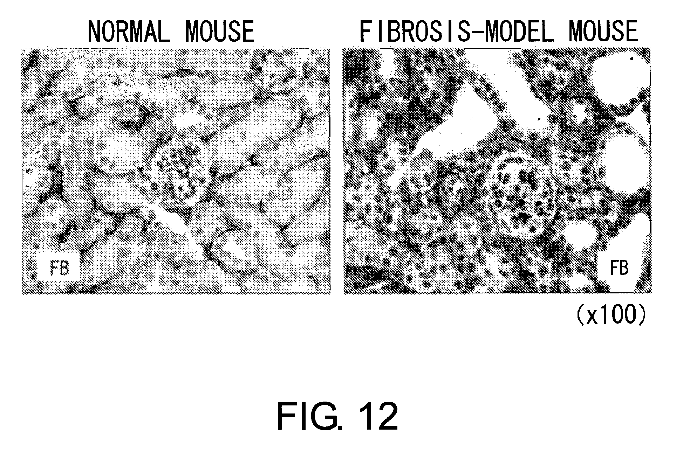

Fig. 12 shows photographs depicting the accumulation of ER-TR7-positive cells in lesions of a fibrosis model. Namely, it shows the result for the kidney tissue of renal fibrosis model mice. Antibody binding was detected by visualizing it as a brown signal.

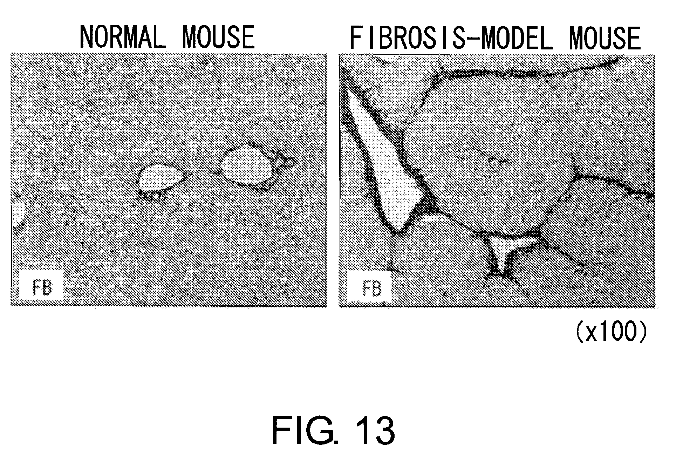

Fig. 13 shows photographs depicting the accumulation of ER-TR7-positive cells in lesions of a fibrosis model. Namely, it shows the result for liver tissue of cirrhosis model mice. Antibody binding was detected by visualizing it as a brown signal.

### Best Mode for Carrying Out the Invention

Chronic fibrotic disease is a general term for the terminal stage of chronic inflammatory conditions that can develop in various organs, and is accompanied by excessive tissue fibrosis. The progression of these diseases results in severe pathological conditions such as organ failure or canceration. The present inventors focused on ER-TR7 as a fibrosis marker, and established an ulcerative colitis model mice to analyze the effect of ER-TR7 administration in detail. The analysis revealed that the administration resulted in improvement of inflammatory symptoms such as reduction of inflammatory activity and suppression of atrophy when compared to wild type colonic mucosa. Specifically, the present inventors discovered that ER-TR7 administration improved fibrosis through neutralization of ER-TR7 antigen, which is closely involved in inflammation as well as fibrosis.

The present invention relates to fibrosis-suppressing agents comprising ER-TR7, or an antibody or compound whose target is an ER-TR7 antigen, as an active ingredient.

The "fibrosis-suppressing agents" of the present invention can effectively suppress tissue fibrosis. Herein, "suppressing tissue fibrosis" means reducing or eliminating fibrotic lesions in fibrotic tissues, or retarding or inhibiting further advancement of fibrosis (suppressing the expansion of fibrotic lesions).

For example, the degree of fibrosis in liver tissues can be evaluated by various methods known to those skilled in the art. In the most fundamental method, fibrosis may be evaluated by histologically observing fibrotic findings in a liver biopsy, for example, images of fibrotic tissues emphasized by special staining (aniline blue stain, trichrome stain, silver stain, and such) in liver biopsy samples. Specifically, evaluation of fibrosis can be achieved, for example, by presenting the degree of hepatic fibrosis in each sample measured by immunohistochemical staining in terms of a fibrosis score according to Dai K, et al., World J Gactroenterol. 2005, 31, 4822-4826; or Hillebrandt S, et al., Nature Genetics 2005, 37, 835-843. Alternatively, the degree of hepatic fibrosis in liver tissues may be evaluated more simply by using hepatic fibrosis markers, such as hyaluronic acid, type I, III, and IV collagens and other collagens, fibroblasts, and macrophages. Simple tests that comprise determining the platelet count, which sensitively reflects the degree of hepatic fibrosis, can also be conveniently used. Alternatively, the degree of hepatic fibrosis can also be roughly estimated by image diagnosis of the liver, such as abdominal ultrasonographic examinations. Alternatively, the degree of hepatic fibrosis can also be evaluated using a measurement device (FibroScan 502, or such) for a non-invasive method that examines hepatic fibrosis based on the transient elastography technology recently developed by EchoSens (France). The degree of suppression of hepatic fibrosis by fibrosis-suppressing agent of the present invention may be determined based on evaluation of the degree of hepatic fibrosis by the methods described above.

The fibrosis-suppressing effect of fibrosis-suppressing agents of the present invention is preferably accompanied by in particular, suppression of at least one of type III collagen elongation, type I collagen deposition, fibroblast accumulation, and macrophage accumulation in body tissues such as skin and internal organ, and more preferably suppression of all of them. The degree of suppression of fibrosis by the fibrosis-suppressing agents of the present invention may be determined using one or more of the suppression effects described above as an indicator.

"ER-TR7" of the present invention is a rat monoclonal antibody against non-lymphocytic cells of mouse thymus, which was produced by Van Vliet *et al.* ER-TR7 is commercially available from several suppliers as an antibody that recognizes connective tissues of various mouse and human organs. "ER-TR7" of the present invention is not limited to commercially available ER-TR7, also includes antibodies which share the same epitope as that recognized by ER-TR7, and antibodies that recognize as an epitope a different portion in ER-TR7 antigen. The species from which ER-TR7 is derived are not particularly limited; however human is preferred. Thus, "ER-TR7" of the present invention also includes polyclonal and monoclonal antibodies that recognize a nonhuman protein equivalent to the ER-TR7 antigen (homolog, ortholog, etc.). In addition, recombinant antibodies prepared by using genetic engineering techniques are included in the "ER-TR7" of the present invention. "Substances that are functionally equivalent" to the ER-TR7 of the present invention include antibodies whose target is the same antigen as ER-TR7. However, the substances are not limited to the above examples. Compounds having the activity of inhibiting or neutralizing the ER-TR7 antigen by binding, are also included.

In the present invention, the "inhibition or neutralization of production and accumulation" of ER-TR7 antigen includes, for example, "inhibition of synthesis", "enhancement of degradation", "inhibition of modification", "demodification", and "elimination" of ER-TR7 antigen activity, but is not limited thereto. The "inhibition or neutralization of production and accumulation" of ER-TR7 antigen means that the content, function, or activity of ER-TR7 antigen is reduced or eliminated as compared to a control. In the present invention, the "inhibition or neutralization of production and accumulation" of ER-TR7 antigen is not particularly limited; however, such preferred substances are "substances with the activity of promoting degradation", "substances with the activity of inhibiting synthesis", "substances with the activity of inhibiting modification", "substances with the activity of promoting demodification" of ER-TR7 antigen, or "substances with the activity of neutralizing" ER-TR7 antigen.

"Expression" includes "transcription" from genes and "translation" into polypeptides. Furthermore, "suppression of degradation" of proteins is also included. The "expression of ER-TR7 antigen proteins" refers to transcription and translation of the genes encoding ER-TR7 antigen proteins, or production of ER-TR7 antigen proteins through transcription and translation. Furthermore, the "function of ER-TR7 antigen proteins" includes, for example, their binding with other cellular components or such. Those skilled in the art can appropriately evaluate (measure) the various above-mentioned functions using general methods. Specifically, the evaluation can be performed using the methods described in the Examples herein below, or using the same methods with appropriate modifications.

The "promotion of degradation" of ER-TR7 antigen may also be an increase in the expression of enzymes that cleave or degrade ER-TR antigen, or of enzymes involved in the cleavage or degradation of antigens. Furthermore, the "promotion of degradation" may be caused by administering a substance that promotes the expression of ER-TR7 antigens.

Preferred embodiments of the substance with an activity of promoting degradation include, for example, compounds selected from the group consisting of:
(a) antibodies that bind to ER-TR7 antigen proteins;
(b) ER-TR7 antigen protein variants that are dominant-negative for ER-TR7 antigen proteins; and
(c) low-molecular-weight compounds that bind to ER-TR7 antigen proteins.

Furthermore, the substance with an activity of promoting degradation include, for example, compounds (nucleic acids) selected from the group consisting of:
(a) antisense nucleic acids against transcripts of the genes encoding ER-TR7 antigen proteins, or portions thereof;
(b) nucleic acids with the ribozyme activity of specifically cleaving transcripts of genes encoding ER-TR7 antigen proteins; and
(c) substances with the activity of using RNAi effect to inhibit the expression of genes encoding ER-TR7 antigen proteins.

"Inhibition of synthesis" of ER-TR7 antigens includes, for example, inhibition of the activities of enzymes involved in the synthesis of ER-TR7 antigens, but is not limited thereto. The inhibition refers to inhibition of any of the processes of ER-TR7 antigen synthesis.

Preferred embodiments of substances with an activity of inhibiting synthesis include, for example, compounds (nucleic acids) selected from the group consisting of:
(a) antibodies that bind to ER-TR7 antigen protein synthetases;
(b) ER-TR7 antigen protein synthetase variants that are dominant-negative for ER-TR7 antigen protein synthetases; and
(c) low-molecular-weight compounds that bind to ER-TR7 antigen protein synthetases.

The substances with the activity of inhibiting synthesis also include, for example, compounds (nucleic acids) selected from the group consisting of:
(a) antisense nucleic acids against transcripts of genes encoding ER-TR7 antigen protein synthetases, or portions thereof;
(b) nucleic acids with the ribozyme activity of specifically cleaving transcripts of the genes encoding ER-TR7 antigen protein synthetases; and
(c) substances with the activity of using RNAi effect to inhibit the expression of genes encoding ER-TR7 antigen protein synthetases.

"Modification" of ER-TR7 antigen refers to post-translational modification of ER-TR7, and includes, for example, addition of sugar chains or lipids, or functional groups such as phosphate or methyl groups.

Preferred embodiments of substances with an activity of inhibiting modification include, for example, compounds (nucleic acids) selected from the group consisting of:
(a) antibodies that bind to ER-TR7 antigen protein modification enzymes;
(b) ER-TR7 antigen protein modification enzyme variants that are dominant negative for ER-TR7 antigen protein modification enzymes; and
(c) low-molecular-weight compounds that bind to ER-TR7 antigen protein modification enzymes.

The "substances with the activity of inhibiting modification" also include compounds selected from the group consisting of:
(a) antisense nucleic acids against transcript of the genes encoding ER-TR7 antigen protein modification enzymes, or portions thereof;
(b) nucleic acids with the ribozyme activity of specifically cleaving transcripts of genes encoding ER-TR7 antigen protein modification enzymes; and
(c) substances with the activity of using RNAi effect to inhibit the expression of genes encoding ER-TR7 antigen protein modification enzymes.

The "demodification" of ER-TR7 antigen refers to a reaction such as hydrolysis to eliminate the post-translational modification that is required for the activity of ER-TR7 antigen.

Preferred embodiments of the "substances with the activity of promoting demodification" include, for example, compounds selected from the group consisting of:
(a) antibodies that bind to ER-TR7 antigen protein demodification enzyme-suppressing proteins;
(b) ER-TR7 antigen protein demodification enzyme-suppressing protein variants which are dominant negative for ER-TR7 antigen protein demodification enzyme-suppressing proteins; and
(c) low-molecular-weight compounds that bind to ER-TR7 antigen protein demodification enzyme-suppressing proteins.

The "substances with the activity of promoting demodification" also include compounds (nucleic acids) selected from the group consisting of:
(a) antisense nucleic acids against transcripts of the genes encoding ER-TR7 antigen protein demodification enzyme-suppressing proteins, or portions thereof;
(b) nucleic acids with the ribozyme activity of specifically cleaving transcripts of genes encoding ER-TR7 antigen protein demodification enzyme-suppressing proteins; and
(c) substances with the activity of using RNAi effect to inhibit the expression of genes encoding ER-TR7 antigen protein demodification enzyme-suppressing proteins.

The "neutralization" of ER-TR7 antigen refers to the suppression of ER-TR7 antigen activity through binding of a compound to the activation site of the ER-TR7 antigen, and such compounds include, for example, ER-TR7 antigen activation-suppressing proteins that bind to the activation site of ER-TR7 antigen.

Preferred embodiments of "substance with the neutralizing activity" include, for example, compounds selected from the group consisting of:
(a) antibodies that bind to ER-TR7 antigens;
(b) ER-TR7 antigen activation-suppressing protein variants that are dominant-negative for ER-TR7 antigen activation-suppressing proteins; and
(c) low-molecular-weight compounds that bind to ER-TR7 antigens.

The "substance with the activity of promoting demodification" also include, for example, compounds (nucleic acids) selected from the group consisting of:
(a) antisense nucleic acids against transcripts of genes encoding ER-TR7 antigen activation-suppressing proteins, or portions thereof;
(b) nucleic acids with the ribozyme activity of specifically cleaving transcripts of the genes encoding ER-TR7 antigen activation-suppressing proteins; and
(c) substances with the activity of using RNAi effect to inhibit the expression of genes encoding ER-TR7 antigen activation-suppressing proteins.

Antibodies that bind to the above-described ER-TR7 antigen protein, synthetase, modification enzyme, demodification enzyme-suppressing protein, ER-TR7 antigen activation-suppressing protein can be prepared by methods known to those skilled in the art. Polyclonal antibodies can be obtained, for example, by the following procedure: small animals such as rabbits are immunized with an above-described natural protein or a recombinant protein expressed in microorganisms as a fusion protein with epitope tags such as GST, or a partial peptide thereof. Sera are obtained from these animals and purified by, for example, ammonium sulfate precipitation, Protein A or G columns, DEAE ion exchange columns, affinity columns coupled with the ER-TR7 antigen protein, synthetase, modification enzyme, demodification enzyme-suppressing protein, ER-TR7 antigen activation-suppressing protein described above, a synthetic peptide, or such, to prepare antibodies. Monoclonal antibodies can be obtained by the following procedure: small animals such as mice are immunized with an above-described ER-TR7 antigen protein, synthetase, modification enzyme, demodification enzyme-suppressing protein, ER-TR7 antigen activation-suppressing protein, or a partial peptide thereof. Spleens are removed from the mice and crushed to isolate cells. The cells are fused with mouse myeloma cells using a reagent such as polyethylene glycol. Clones producing antibodies that bind to an above-described ER-TR7 antigen protein, synthetase, modification enzyme, demodification enzyme-suppressing protein, ER-TR7 antigen activation-suppressing protein are selected from among the resulting fused cells (hybridomas). The obtained hybridomas are then transplanted in the peritoneal cavities of mice, and ascites collected. The obtained monoclonal antibodies can be purified by, for example, ammonium sulfate precipitation, Protein A or G columns, DEAE ion exchange columns, affinity columns coupled with the ER-TR7 antigen protein, synthetase, modification enzyme, demodification enzyme-suppressing protein, ER-TR7 antigen activation-suppressing protein described above, or a synthetic peptide, or such.

The antibodies of the present invention are not particularly limited as long as they bind to an above-described ER-TR7 antigen protein, synthetase, modification enzyme, demodification enzyme-suppressing protein, ER-TR7 antigen activation-suppressing protein of the present invention. The antibodies of the present invention may be human antibodies, humanized antibodies created by gene recombination, fragments or modified products of such antibodies, in addition to the polyclonal and monoclonal antibodies described above.

The proteins of the present invention used as sensitizing antigens to prepare antibodies are not limited in terms of the animal species from which the proteins are derived. However, the proteins are preferably derived from mammals, for example, mice and humans. Human-derived proteins are particularly preferred.

In the present invention, the proteins to be used as sensitizing antigens may be whole proteins or partial peptides thereof. Such partial peptides of the proteins include, for example, amino-terminal fragments and carboxyl-terminal fragments of the proteins. Herein, "antibodies" refer to antibodies that react with a full-length protein or fragment thereof.

In addition to immunizing nonhuman animals with antigens to obtain the above hybridomas, human lymphocytes, for example, EB virus-infected human lymphocytes, can be sensitized *in vitro* with the proteins or with cells expressing the proteins, or with lysates thereof, and the sensitized lymphocytes can be fused with human-derived myeloma cells with the ability to divide permanently, for example, U266, to obtain hybridomas that produce desired human antibodies with binding activity to the proteins.

It is expected that antibodies against the above-described ER-TR7 antigen proteins, synthetases, modification enzymes, demodification enzyme-suppressing proteins, ER-TR7 antigen activation-suppressing proteins of the present invention exhibit the effect of inhibiting protein expression or function by binding to the proteins. When using the prepared antibodies for human administration (antibody therapy), the antibodies are preferably human or humanized antibodies in order to reduce immunogenicity.

In addition, included in the present invention are methods for producing fibrosis-suppressing agents comprising as an active ingredient an antibody that binds to an ER-TR7 antigen protein, which comprise the step of preparing the antibody by using the antigen protein.

Furthermore, in the present invention, low-molecular-weight substances (low-molecular-weight compounds) that bind to the above-described ER-TR7 antigen proteins, synthetases, modification enzymes, demodification enzyme-suppressing proteins, ER-TR7 antigen activation-suppressing proteins are also included in the substances capable of inhibiting the function of the above-described ER-TR7 antigen proteins, synthetases, modification enzymes, demodification enzyme-suppressing proteins, ER-TR7 antigen activation-suppressing proteins. Such low-molecular-weight substances may be natural or artificial compounds. In general, the compounds can be produced or obtained by methods known to those skilled in the art. In addition, the substances of the present invention capable of inhibiting the expression or function of the above-described ER-TR7 antigen proteins, synthetases, modification enzymes, demodification enzyme-suppressing proteins, ER-TR7 antigen activation-suppressing proteins include dominant-negative mutants (dominant-negative proteins) for the above-described ER-TR7 antigen proteins, synthetases, modification enzymes, demodification enzyme-suppressing proteins, ER-TR7 antigen activation-suppressing proteins.

"A protein variant that is dominant negative for an ER-TR7 antigen protein, synthetase, modification enzyme, demodification enzyme-suppressing protein, or ER-TR7 antigen activation-suppressing protein" refers to a protein that, when expressed, has the function of reducing or eliminating the activity of the endogenous wild type protein.

In the present invention, "nucleic acids" refer to both RNAs and DNAs. Chemically synthesized nucleic acid analogs, such as so-called "PNAs" (peptide nucleic acids) or Morpholino antisense oligos, are also included in the nucleic acids of the present invention. PNAs are nucleic acids in which the fundamental backbone structure of nucleic acids, the pentose-phosphate backbone, is replaced by a polyamide backbone with glycine units, and Morpholino antisense oligos are nucleic acids in which the pentose-phosphate backbone is replaced by a morpholino backbone. PNAs and morpholino antisense oligos have a three-dimensional structure quite similar to that of nucleic acids.

Methods for suppressing the expression of specific endogenous genes using antisense technology are well known to those skilled in the art. There are a number of causes for the action of antisense nucleic acids in suppressing target gene expression, including:
inhibition of transcription initiation by triplex formation;
transcription inhibition by hybrid formation at a site with a local open loop structure generated by an RNA polymerase;
transcription inhibition by hybrid formation with the RNA being synthesized;
splicing inhibition by hybrid formation at an intron-exon junction;
splicing inhibition by hybrid formation at the site of spliceosome formation;
inhibition of transport from the nucleus to the cytoplasm by hybrid formation with mRNA;
translation initiation inhibition by hybrid formation at the capping site or poly(A) addition site;
inhibition of translation initiation by hybrid formation at the translation initiation factor binding site;
inhibition of translation by hybrid formation at the ribosome binding site adjacent to the initiation codon;
inhibition of peptide chain elongation by hybrid formation in the translational region of mRNA or at the polysome binding site of mRNA; and
inhibition of gene expression by hybrid formation at the protein-nucleic acid interaction sites (Hirashima and Inoue, Shin Seikagaku Jikken Koza 2 (New Courses in Experimental Biochemistry 2), Kakusan (Nucleic Acids) IV: "Idenshi no Fukusei to Hatsugen (Gene replication and expression)", Ed. The Japanese Biochemical Society, Tokyo Kagakudojin, 1993, pp. 319-347). There are causes for the action of antisense RNA in suppressing target gene expression includes inhibition of gene expression by RNAi effect of double strand RNA formation by hybrid formation with mRNA, and such. Thus, antisense nucleic acids inhibit the expression of target genes by inhibiting various processes, such as transcription, splicing, and
translation.

The antisense nucleic acids used in the present invention may inhibit the expression and/or function of genes encoding any of the ER-TR7 antigen proteins, synthetases, modification enzymes, demodification enzyme-suppressing proteins, ER-TR7 antigen activation-suppressing proteins described above, based on any of the actions described above. In one embodiment, antisense sequences designed to be complementary to an untranslated region adjacent to the 5' end of an mRNA for a gene encoding an above-described ER-TR7 antigen protein, synthetase, modification enzyme, demodification enzyme-suppressing protein, ER-TR7 antigen activation-suppressing protein may be effective for inhibiting translation of the gene. Sequences complementary to a coding region or 3'-untranslated region can also be used. Thus, the antisense nucleic acids to be used in the present invention include not only nucleic acids comprising sequences antisense to the coding regions, but also nucleic acids comprising sequences antisense to untranslated regions of genes encoding the above-described ER-TR7 antigen proteins, synthetases, modification enzymes, demodification enzyme-suppressing proteins, ER-TR7 antigen activation-suppressing proteins. Such antisense nucleic acids to be used are linked downstream of adequate promoters and are preferably linked with transcription termination signals on the 3' side. Nucleic acids thus prepared can be introduced into desired animals (cells) using known methods. The sequences of the antisense nucleic acids are preferably complementary to a gene or portion thereof encoding an ER-TR7 antigen protein, synthetase, modification enzyme, demodification enzyme inhibiting protein, ER-TR7 antigen activation inhibiting protein that is endogenous to the animals (cells) to be transformed with them. However, the sequences need not be perfectly complementary, as long as the antisense nucleic acids can effectively suppress expression of a gene. The transcribed RNAs preferably have 90% or higher, and most preferably 95% or higher complementarity to target gene. To effectively inhibit target gene expression using antisense nucleic acids, the antisense nucleic acids are preferably at least 15 nucleotides long, and less than 25 nucleotides long. However, the lengths of the antisense nucleic acids of the present invention are not limited to the lengths mentioned above, and they may be 100 nucleotides or more, or 500 nucleotides or more.

Expression of the above-mentioned genes encoding ER-TR7 antigen proteins, synthetases, modification enzymes, demodification enzyme-suppressing proteins, ER-TR7 antigen activation-suppressing proteins can also be inhibited using ribozymes or ribozyme-encoding DNAs. Ribozymes refer to RNA molecules with catalytic activity. There are various ribozymes with different activities. Among others, studies that focused on ribozymes functioning as RNA-cleaving enzymes have enabled the design of ribozymes that cleave RNAs in a site-specific manner. Some ribozymes have 400 or more nucleotides, such as group I intron type ribozymes and M1 RNA, which is comprised by RNase P, but others, called hammerhead and hairpin ribozymes, have a catalytic domain of about 40 nucleotides (Koizumi, M. and Otsuka, E., Tanpakushitsu Kakusan Koso (Protein, Nucleic Acid, and Enzyme) 1990, 35, 2191).

For example, the autocatalytic domain of a hammerhead ribozyme cleaves the sequence G13U14C15 at the 3' side of C15. Base pairing between U14 and A9 has been shown to be essential for this activity, and the sequence can be cleaved when C15 is substituted with A15 or U15 (Koizumi, M. et al., FEBS Lett. 1988, 239, 285; Koizumi, M. and Otsuka, E., Tanpakushitsu Kakusan Koso (Protein, Nucleic Acid, and Enzyme) 1990, 35, 2191; and Koizumi, M. et al., Nucl. Acids Res. 1989, 17, 7059).

In addition, hairpin ribozymes are also useful for the purposes of the present invention. Such ribozymes are found in, for example, the minus strand of satellite RNAs of tobacco ringspot viruses (Buzayan, J.M., Nature 1986, 323, 349). It has been shown that target-specific RNA-cleaving ribozymes can also be created from hairpin ribozymes (Kikuchi, Y. and Sasaki, N., Nucl Acids Res. 1991, 19, 6751; and Kikuchi, Y. Kagaku to Seibutsu (Chemistry and Biology) 1992, 30, 112). Thus, the expression of the above-described genes encoding ER-TR7 antigen proteins, synthetases, modification enzymes, demodification enzyme-suppressing proteins, ER-TR7 antigen activation-suppressing proteins can be inhibited by using ribozymes to specifically cleave the gene transcripts.

The expression of endogenous genes can also be suppressed by RNA interference (hereinafter abbreviated as "RNAi"), using double-stranded RNAs comprising a sequence the same as or similar to a target gene sequence.

A great many disease-related genes have been rapidly identified since the entire human genome nucleotide sequence was revealed upon the recent completion of the genome project, and currently specific gene-targeted therapies and drugs are being actively developed. Of these, the application to gene therapy of small interfering RNAs (siRNAs), which produce the effect of specific post-transcriptional suppression, has been drawing attention. RNAi is a technology currently drawing attention in which double-stranded RNAs (dsRNAs) incorporated into cells suppress the expression of genes with sequences homologous to the dsRNAs. In mammalian cells, RNAi can be induced using dsRNAs and has many advantages: compared to knockout mice, RNAi has a stable effect, simple experiments, low costs, and so on.

RNAi is a phenomenon where an mRNA comprising a base sequence complementary to a double-stranded RNA is degraded. RNAi is a method based on this phenomenon, in which the expression of an arbitrary gene is suppressed by artificially introducing a 21- to 23-mer double-stranded RNA (siRNA). In 1998, Fire *et al.* discovered using *C. elegans* that double-stranded RNA silences genes in a sequence-specific manner (Fire, A. et al., Nature 1998, 391, 806-811). After elucidating the underlying mechanism of mRNA cleavage by 21- to 23-mer processed double-stranded RNA (Zamore PD. et al., Cell 2000, 101, 25-33), identifying RNA-induced silencing complex (RISC) (Hammond, S.M. et al., Science 2001, 293, 1146-1150), and cloning Dicer (Bernstein, E. et al., Nature, 409, 363-366), Elbashir *et al.* demonstrated in 2001 that siRNA could also suppress expression in a sequence-specific manner in mammalian cells (Elbashir SM. et al., Nature 2001, 411, 494-498). Thus, application of RNAi to gene therapy is highly expected.

Nucleic acids with inhibitory activity based on RNAi effect are generally referred to as siRNAs or shRNAs. RNAi is a phenomenon in which, when cells or such are introduced with short double-stranded RNAs (hereinafter abbreviated as "dsRNAs") comprising sense RNAs that comprise sequences homologous to the mRNAs of a target gene, and antisense RNAs that comprise sequences homologous a sequence complementary thereto, the dsRNAs bind specifically and selectively to the target gene mRNAs, induce their disruption, and cleave the gene transcript, thereby effectively inhibiting (suppressing) target gene expression. For example, when dsRNAs are introduced into cells, the expression of genes with sequences homologous to the RNAs is suppressed (the genes are knocked down). As described above, RNAi can suppress the expression of target genes, and is thus drawing attention as a method applicable to gene therapy, or as a simple gene knockout method replacing conventional methods of gene disruption, which are based on complicated and inefficient homologous recombination. The RNAs to be used in RNAi are not necessarily perfectly identical to the genes or portions thereof that encode an above-described ER-TR7 antigen protein, synthetase, modification enzyme, demodification enzyme-suppressing protein, ER-TR7 antigen activation-suppressing protein; however, the RNAs are preferably perfectly homologous to the genes or portions thereof.

The targets of the siRNAs to be designed are not particularly limited, as long as they are genes encoding an above-described ER-TR7 antigen protein, synthetase, modification enzyme, demodification enzyme inhibiting protein, ER-TR7 antigen activation inhibiting protein. Any region of the gene can be a candidate for a target.

The siRNAs can be introduced into cells by adopting methods that comprise annealing two RNA strands or such. The double-stranded RNA described above may be closed at one end with a hairpin structure (shRNAs). shRNAs refer to short hairpin RNAs, which are RNA molecules with a stem-loop structure, since a portion of the single strand constitutes a strand complementary to another portion. Thus, molecules capable of forming an intramolecular double-stranded RNA are also included in the siRNAs of the present invention.

In a preferred embodiment of the present invention, RNAs (siRNAs) that have an RNAi effect and thus can suppress the genes encoding the above-described ER-TR7 antigen proteins, synthetases, modification enzymes, demodification enzyme-suppressing proteins, or ER-TR7 antigen activation-suppressing proteins are included in the siRNAs of the present invention. For example, even double-stranded RNAs with a structure having a deletion or addition of one or a small number of bases are included, as long as they have the function of suppressing the expression of such genes.

Some details of the RNAi mechanism still remain poorly understood, but it is known that an enzyme called "DICER" (a member of the RNase III nuclease family) binds to a double-stranded RNA and degrades it in to small fragments, called "siRNAs". The double-stranded RNAs of the present invention that have RNAi effect include such double-stranded RNAs prior to being degraded by DICER. Specifically, since even long RNAs that have no RNAi effect when intact can be degraded into siRNAs which have RNAi effect in cells, the length of the double-stranded RNAs of the present invention is not particularly limited.

For example, long double-stranded RNAs covering the full-length or near full-length mRNA of a gene encoding an above-described ER-TR7 antigen protein, synthetase, modification enzyme, demodification enzyme-suppressing protein, ER-TR7 antigen activation-suppressing protein can be pre-digested, for example, by DICER, and then the degradation products can be used as agents of the present invention. These degradation products are expected to contain double-stranded RNA molecules with RNAi effect. With this method, it is not necessary to specifically select the regions expected to have RNAi effect. In other words, it is not necessary to accurately determine regions with RNAi effect in the mRNAs of the genes described above.

The siRNAs of the present invention are not necessarily single pairs of double-stranded RNAs directed to target sequences, but may be mixtures of multiple double-stranded RNAs directed to regions that cover the target sequence. The siRNAs of the present invention include so-called "siRNA cocktails".

All nucleotides in the siRNAs of the present invention do not necessarily need to be ribonucleotides (RNAs). Specifically, one or more of the ribonucleotides constituting the siRNAs of the present invention may be replaced with corresponding deoxyribonucleotides. The term "corresponding" means that although the sugar moieties are structurally differently, the nucleotide residues (adenine, thymine (uracil), guanine, or cytosine) are the same. For example, deoxyribonucleotides corresponding to ribonucleotides with adenine refer to deoxyribonucleotides with adenine. The term "or more" described above is not particularly limited, but preferably refers to a small number of about two to five ribonucleotides.

Furthermore, DNAs (vectors) that are capable of expressing the above-described RNAs of the present invention are also included in preferred embodiments of the above-described compounds that can suppress the expression of genes encoding ER-TR7 antigen proteins, synthetases, modification enzymes, demodification enzyme-suppressing proteins, or ER-TR7 antigen activation-suppressing proteins of the present invention. The DNAs that are capable of expressing the above-described double-stranded RNAs of the present invention are, for example, DNAs having a structure in which a promoter(s) is linked to DNA encoding one strand of the double-stranded RNA and to DNA encoding the other so as to express both of the RNAs. Alternatively, such DNAs may be those in which a target sequence has been flanked by appropriate sequences and inserted as a palindromic structure downstream of an appropriate promoter. This yields an shRNA described above.

Furthermore, the expression-inhibitory substances of the present invention include compounds that inhibit the expression of genes encoding the above-described ER-TR7 antigen proteins, synthetases, modification enzymes, demodification enzyme-suppressing proteins, or ER-TR7 antigen activation-suppressing proteins, by binding to transcription factors that bind to the expression regulatory regions of genes encoding the above-described ER-TR7 antigen proteins, synthetases, modification enzymes, demodification enzyme-suppressing proteins, or ER-TR7 antigen activation-suppressing proteins.

The present invention also relates to methods for suppressing fibrosis in body tissues such as skin and organs of subjects, in which "fibrosis-suppressing agents" of the present invention are administered to the subjects.

Furthermore, the "fibrosis-suppressing agents" of the present invention can be advantageously used to treat or prevent fibrotic diseases (diseases associated with excessive tissue fibrosis), because they can efficiently suppress fibrosis of body tissues such as skin and organs. In the present invention, there is no limitation to the type of fibrotic disease. The "fibrotic diseases" in the present invention is not particularly limited, and specifically include, for example, elastosis, scleroderma, chronic peritonitis, and retroperitoneal fibrosis in integumentary and epithelial tissues such as skin;
polymyositis, dermatomyositis, polyarteritis nodosa, soft tissue fibrosis, chronic rheumatoid arthritis, palmar fibromatosis, tendinitis, tenovaginitis, Achilles tendinitis, mycetoma pedis, and such in supportive tissues such as connective tissues and muscles;
myelofibrosis, hypersplenism, vasculitis, bradyarrhythmia, arteriosclerosis, obstructive thrombotic angiitis, nodular fibrosis, angina pectoris, dilated congestive cardiomyopathy, heart failure, restrictive cardiomyopathy, diffuse nonobstructive cardiomyopathy, obstructive cardiomyopathy, cor pulmonale, mitral stenosis, aortic valve stenosis, chronic pericarditis, endocardial fibrosis, endomyocardial fibrosis, and such in blood tissues and vascular system such as bone marrow and heart;
chronic pancreatitis, Crohn's disease, ulcerative colitis, alcoholic hepatitis, chronic hepatitis B, chronic hepatitis C, Wilson's disease, cirrhosis, viral hepatitis, Gaucher's disease, glycogen storage disease, alpha 1-antitrypsin deficiency, hemochromatosis, tyrosinemia, levulosemia, galactosemia, Zellweger syndrome, congenital hepatic fibrosis, portal hypertension, hepatic granulomatosis, Budd-Chiari syndrome, primary sclerosing cholangitis, fatty liver, nonalcoholic hepatitis, hepatic fibrosis, congenital hepatic fibrosis, alcoholic cirrhosis, viral cirrhosis, parasitic cirrhosis, toxic cirrhosis, trophopathic cirrhosis, congestive cirrhosis, hepatic sclerosis, Charcot's cirrhosis, Todd's cirrhosis, secondary biliary cirrhosis, unilobar cirrhosis, cirrhosis resulting from chronic nonsuppurative destructive cholangitis, obstructive cirrhosis, cholangiolitic cirrhosis, biliary cirrhosis, atrophic cirrhosis, postnecrotic cirrhosis, posthepatitic cirrhosis, nodular cirrhosis of the liver, mixed cirrhosis, micronodular cirrhosis, compensatory cirrhosis, decompensated cirrhosis, macronodular cirrhosis, septal cirrhosis, cryptogenic cirrhosis, periportal cirrhosis, portal cirrhosis, primary biliary cirrhosis, and such in the gastrointestinal system such as liver; coccidioidomycosis, blastomycosis, allergic bronchopulmonary aspergillosis, Goodpasture's syndrome, pulmonary fibrosis associated with adult respiratory distress syndrome, chronic obstructive pulmonary disease, pulmonary atelectasis, pneumonia, chalicosis, asbestosis, hypersensitivity pneumonitis, idiopathic pulmonary fibrosis, lymphocytic interstitial pneumonia, Langerhans-cell granulomatosis, cystic fibrosis, pustular fibrosis, pulmonary fibrosis, idiopathic pulmonary fibrosis, fibrosing pulmonary alveolitis, interstitial fibrosis, diffuse pulmonary fibrosis, chronic interstitial pneumonia, bronchiectasis, bronchiolar fibrosis, peribronchial fibrosis, pleural fibrosis, and such in the respiratory system such as lung; male hypogonadism, myotonic dystrophy, fibrosis such as associated with Peyronie's disease, chronic tubulointerstitial nephritis, autosomal recessive cystic kidney, myeloma kidney, hydronephrosis, rapidly progressive glomerulonephritis, nephrotoxic diseases, xanthogranulomatous pyelonephritis, sickle cell nephropathy, nephrogenic diabetes insipidus, autosomal dominant polycystic kidney disease, chronic glomerular nephritis, IgA nephropathy, renal sclerosis, focal glomerulosclerosis, membranous nephritis, membranoproliferative glomerulonephritis, chronic pyelonephritis, renal amyloidosis, polycystic kidney disease, retroperitoneal fibrosis, pathology in the kidney associated with a connective tissue disease such as lupus nephritis, diabetic nephropathy, chronic prostatitis, and urocystitis associated with schistosomiasisin the urogenital system such as kidney;
fibrotic breast disease, mammary fibroadenoma, and such;
congenital torticollis, ankylosing spondylitis, spinal cord disorders such as neurofibroma and neurological dysfunction after spinal cord injury, and cranial nerve diseases such as Parkinson's disease and Alzheimer's disease in the nervous system such as spine;
retrolental fibrosis and proliferative retinopathy in the eyeball; and sarcoidosis that develops systemic involvement, fibrosis and systemic scleroderma associated with systemic lupus erythematosus, polymyositis, dermatomyositis, and such. However, in the present invention, the "fibrotic disease" is not limited thereto, and includes diseases caused by fibrosis in each body tissue such as skin and organs.

The "fibrosis-suppressing agents" of the present invention can also be referred to as "therapeutic agents for fibrosis", "fibrosis-improving agents", "antifibrotic agents", or the like. Meanwhile, the "suppressing agents" of the present invention can also be referred to as "pharmaceutical agents", "pharmaceutical compositions", "therapeutic medicines", or the like.

The "treatments" of the present invention also comprise preventive effects, ameliorating effects, or such that can suppress the onset of fibrosis in advance. The treatments are not necessarily limited to those producing a perfect therapeutic effect on tissues developing fibrosis, and the effects may be partial.

According to the present invention, fibrosis of body tissue in a subject can be effectively suppressed by administering a "fibrosis-suppressing agent" of the present invention to the subject. There is no limitation on the administration route of the "fibrosis-suppressing agents"; however, parenteral route is preferred. Preferred examples of such parenteral routes include intravenous, intraarterial, intraperitoneal, and subcutaneous routes. The "fibrosis-suppressing agents" of the present invention may be administered systemically or locally (for example, administered directly to fibrotic liver tissues). The administered dose varies depending on the patient's weight and age, and the administration method or such; however, those skilled in the art (medical practitioners, veterinarians, pharmacists, and the like) can appropriately select a suitable dose.

Preferred subjects to be administered with the "fibrosis-suppressing agents" of the present invention are mammals, including humans, domestic animals, pets, and experimental animals. In particular, mammals (patients) with fibrotic diseases, for example, cirrhosis and pulmonary fibrosis, are preferred subjects of the present invention.

The pharmaceutical agents of the present invention may be administered orally or parenterally as pharmaceutical compositions appropriately comprising pharmaceutically acceptable (pharmaceutically inactive) carriers, excipients, and/or diluents. Such carriers, excipients, and/or diluents of the present invention include, but are not limited to, for example, water, physiological saline, phosphate buffered saline, polyvinyl alcohol, polyvinylpyrrolidone, carboxylvinyl polymer, sodium alginate, water-soluble dextran, pectin, xanthan gum, gum Arabic, gelatin, agar, glycerin, propylene glycol, polyethylene glycol, vaseline, paraffin, stearyl alcohol, stearic acid, human serum albumin, mannitol, sorbitol, and lactose. The pharmaceutical compositions of the present invention may further comprise additives, such as preservatives. The pharmaceutical compositions of the present invention may further comprise other pharmacological ingredients.

The pharmaceutical composition of the present invention may be provided as oral or parenteral preparations; however they are more preferably provided as parenteral preparations. Preferred parenteral preparations are liquid preparations such as liquids and suspensions. Injections or drops are especially preferred.

Furthermore, the present invention provides methods for treating or preventing fibrotic diseases, which comprise the step of administering agents of the present invention to individuals (for example, patients with fibrotic diseases, etc.).

The present invention also relates to the use of the above-described ER-TR7 or functionally equivalent substances in producing the agents of the present invention (fibrosis-suppressing agents, agents for treating or preventing fibrotic diseases, and such).

All publications, patents, and patent applications cited herein are incorporated herein by reference in their entirety.

### Examples

Hereinbelow, the present invention will be specifically described with reference to Examples, but the technical scope of the present invention is not to be construed as being limited thereto.

### [Example 1] Therapeutic effect of ER-TR7 in cirrhosis model mice: clinical presentation

A mixture of carbon tetrachloride (10 µl; Sigma-Aldrich) with 90 µl of mineral oil (Sigma-Aldrich) was administered into the peritoneal cavities of C57BL/6J mice (female, five to six weeks old; CLEA Japan Inc.) twice a week for four weeks (seven times) to induce hepatic fibrosis. Then, carbon tetrachloride was additionally administered twice a week for two weeks (11 times in all) to induce cirrhosis. The carbon tetrachloride-induced hepatic fibrosis model has excellent reproducibility, and is thus widely used as an experimental cirrhosis model (Sakaida I., et al., Hepatology, 40: 1304-1311, 2004). ER-TR7 (10 µl in 200 µl of PBS) was administered to the cirrhosis-induced mice twice a week for two weeks (four times). These mice were used as an ER-TR7-treated group. Furthermore, mice administered with PBS alone in the same way were used as an untreated group, while cirrhosis-uninduced mice, which were not administered with carbon tetrachloride, were used as a control group. Mice of each group were sacrificed to collect their livers.

Portions of second lobes from the collected livers were embedded in the OCT compound (Miles), an embedding medium for cryosectioning. Cryoblocks were prepared using liquid nitrogen, and then sliced into 6 µm-thick sections using Cryostat (Microm).

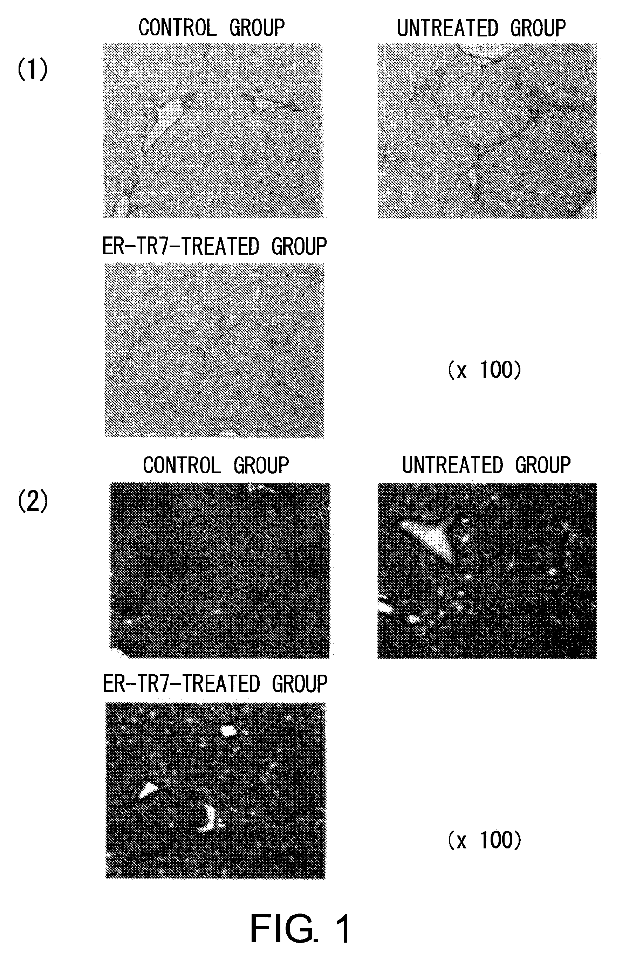

The resulting sections were fixed with acetone (Wako Pure Chemical Industries Ltd.) for ten minutes, and then washed with phosphate buffer. Next, anti-type IV collagen antibody (rabbit serum, 2,000 times dilution; LSL) was added as the primary antibody, and the sections were incubated at room temperature for one hour. Then, the secondary antibody reaction was conducted using peroxidase-labeled anti-rabbit antibody (200 times dilution; MP biomedicals), followed by addition of DAB substrate (Nichirei) (Fig. 1(1)).

Furthermore, the resulting sections were fixed with Bouin' solution (Sigma-Aldrich), and then nuclear staining was carried out using Weigert's Iron Hematoxyline Set (Sigma-Aldrich) according to the instruction manual. After washing with water, the sections were stained with Trichrome Masson (Sigma-Aldrich) according to the instruction manual (Fig. 1(2)). The resulting samples were observed under a light microscope (LEICA Microsystems).

Some examples of the obtained staining images of liver tissues are shown in Fig. 1. The respective stains showed that the images of the ER-TR7-treated group were similar to those of the control group. This suggests that the fibrotic conditions have been improved as compared to the untreated group. Specifically, the ER-TR7 treatment was demonstrated to relieve the fibrosis symptoms.

### [Example 2] Therapeutic effect of ER-TR7 in cirrhosis model mice : determination of expression levels of fibrosis markers

A mixture of carbon tetrachloride (10 µl; Sigma-Aldrich) with 90 µl of mineral oil (Sigma-Aldrich) was administered into the peritoneal cavities of C57BL/6J mice (female, five to six weeks old; CLEA Japan Inc.) twice a week for four weeks (seven times) to induce hepatic fibrosis. Then, carbon tetrachloride was additionally administered twice a week for two weeks (11 times in all) to induce cirrhosis. The carbon tetrachloride-induced hepatic fibrosis model has excellent reproducibility, and is thus widely used as an experimental cirrhosis model (Sakaida I., et al., Hepatology, 40: 1304-1311, 2004). ER-TR7 (10 µl in 200 µl of PBS) was administered to the cirrhosis-induced mice twice a week for two weeks (four times). These mice were used as an ER-TR7-treated group. Furthermore, mice administered with PBS alone in the same way were used as an untreated group, while cirrhosis-uninduced mice, which were not administered with carbon tetrachloride, were used as a control group. Mice of each group were sacrificed to collect their livers.

Some portions were excised from the collected livers, transferred into 1.5-ml tubes, and frozen in liquid nitrogen. 1 ml of RNA-Bee (TEL-TEST, Inc.) per 50 mg of tissue was added. The tissue was homogenized, and 200 µl of chloroform (Sigma-Aldrich) was added to the resulting suspension. The mixture was gently mixed and then cooled on ice for about five minutes, and centrifuged in a centrifuge (Centrifuge 5417R; Eppendorf) at 12,000 rpm and 4°C for 15 minutes. After centrifugation, 500 µl of the supernatant was transferred to a fresh 1.5-ml tube, and an equal volume of isopropanol (500 µl; Sigma-Aldrich) was added thereto. The solution was mixed, and then 1 µl of glycogen (Invitrogen) was added thereto. The mixture was cooled on ice for 15 minutes, and then centrifuged at 12,000 rpm and 4°C for 15 minutes. Next, RNA precipitate obtained after washing three times with 1,000 µl of 75% ethanol (Sigma-Aldrich) was air-dried for 30 minutes to one hour, and then dissolved in Otsuka distilled water (Otsuka Pharmaceutical Co., Ltd). The solution was 100 times diluted with Otsuka distilled water. The RNA concentrations of extracted samples in UV plates (Corning Costar) were determined using a plate reader (POWER Wave XS; BIO-TEK). The concentrations of the obtained RNA samples were adjusted to 500 ng/20 µl. The samples were heated at 68°C for three minutes in a BLOCK INCUBATOR (ASTEC), and cooled on ice for ten minutes. After cooling on ice, 80 µl of RT PreMix solution (composition: 18.64 µl of 25 mg MgCl₂ (Invitrogen), 20 µl of 5x Buffer (Invitrogen), 6.6 µl of 0.1 M DTT (Invitrogen), 10 µl of 10 mM dNTP mix (Invitrogen), 2 µl of Rnase Inhibitor (Invitrogen), 1.2 µl of MMLV Reverse Transcriptase (Invitrogen), 2 µl of Random primer (Invitrogen), and sterile distilled water (Otsuka distilled water; Otsuka Pharmaceutical Co., Ltd.)), which had been prepared in advance, was added to the samples. The mixtures were heated in a BLOCK INCUBATOR at 42°C for one hour and at 99°C for five minutes, and then cooled on ice. cDNAs (100 µl) were prepared by the procedure described above.

PCR was carried out using the prepared cDNAs in the following composition. 1 µl of cDNA was mixed with 12.5 µl of SYBR Premix EX Taq (TAKARA), 11.3 µl of sterile distilled water (Otsuka distilled water), and 0.1 µl of primers (50 pmol/µl; Table 1). The resulting mixtures were reacted in Real-time PCR Dice (TAKARA) with 40 cycles of 95°C for 5 seconds and 60°C for 30 seconds. After reaction, the expression level of each fibrosis marker gene was determined relative to the expression levels of 36B4 gene and GAPDH gene as an internal standard.

**[Table 1]**

| |
|---|
| NM_007393 |
| Mus musculus actin, beta, cytoplasmic (Actb), mRNA (β-actin) |
| F: CATCCGTAAAGACCTCTATGCCAAC (SEQ ID NO: 1) |
| R: ATGGAGCCACCGATCCACA (SEQ ID NO: 2) |
| NM_008084 |
| Mus musculus glyceraldehyde-3-phosphate dehydrogenase, mRNA (GAPDH) |
| F: AAATGGTGAAGGTCGGTGTG(SEQ ID NO: 3) |
| R: TGAAGGGGTCGTTGATGG(SEQ ID NO: 4) |
| NM_007475 |
| Mus musculus acidic ribosomal phosphoprotein P0 (Arbp), mRNA (36B4) |
| F: TTCCAGGCTTTGGGCATCA (SEQ ID NO: 5) |
| R: ATGTTCAGCATGTTCAGCAGTGTG (SEQ ID NO: 6) |
| NM_013693 |
| Mus musculus tumor necrosis factor (Tnf), mRNA (TNF-α) |
| F: CAGGAGGGAGAACAGAAACTCCA(SEQ ID NO: 7) |
| R: CCTGGTTGGCTGCTTGCTT (SEQ ID NO: 8) |
| NM_007743 |
| Mus musculus procollagen, type I, alpha 2 (Col1a2), mRNA (Type 1 collagen) |
| F: ACCCGATGGCAACAATGGA(SEQ ID NO: 9) |
| R: ACCAGCAGGGCCTTGTTCAC (SEQ ID NO: 10) |
| NM_007392 |
| Mus musculus actin, alpha 2, smooth muscle, aorta (Acta2), mRNA (α-SMA) |
| F: GAGCATCCGACACTGCTGACA (SEQ ID NO: 11) |
| R: AGCACAGCCTGAATAGCCACATAC (SEQ ID NO: 12) |

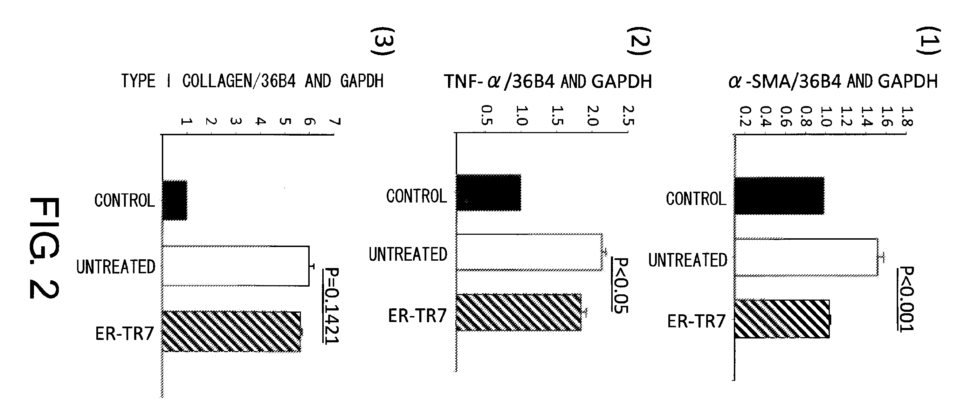

The result showed that the expression levels of all tested genes were reduced in the ER-TR7-treated group when compared to the control group. This suggests that ER-TR7 administration results in suppression of fibrosis (Fig. 2).

### [Example 3] Therapeutic effect of ER-TR7 in ulcerative colitis model mice: macroscopic features

The ulcerative colitis model mice were prepared by allowing C57BL/6J mice (female, seven weeks old; CLEA Japan Inc.) to freely drink high-concentration chlorine water containing 3% dextran sulfate sodium (DSS; Wako Pure Chemical Industries Ltd.) for seven days. At the same time when the mice were fed with 3% DSS water, 10 µl of ER-TR7 (0.4 mg/ml; BMA Biomedicals) premixed with 190 µl physiological saline (Otsuka Pharmaceutical Co., Ltd.), or 200 µl of physiological saline alone was injected into the peritoneal cavities of the mice. The groups of mice treated as described above were named the ER-TR7 group and control group. The mice were reared for eight days while giving 3% DSS water. Then, the mice in each group were sacrificed, and their large intestines were collected to determine their lengths.

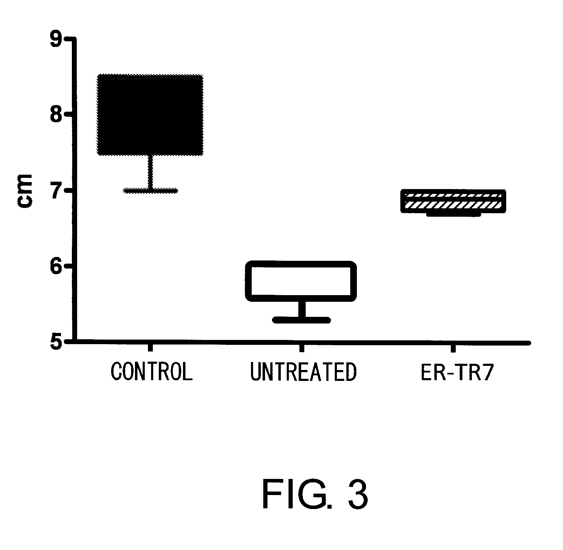

The result showed that the length of the intestines was significantly conserved in the ER-TR7-administered group as compared with the control group (p<0.05; t test). This suggests that ER-TR7 suppresses the atrophy of the large intestine due to fibrous degeneration (Fig. 3).

### [Example 4] Therapeutic effect of ER-TR7 in ulcerative colitis model mice: determination of expression levels of fibrosis marker genes

The ulcerative colitis model mice were prepared by allowing C57BL/6J mice (female, seven weeks old; CLEA Japan Inc.) to freely drink high-concentration chlorine water containing 3% dextran sulfate sodium (DSS; Wako Pure Chemical Industries Ltd.) for seven days. At the same time when the mice were fed with 3% DSS water, 10 µl of ER-TR7 (0.4 mg/ml; BMA Biomedicals) premixed with 190 µl physiological saline (Otsuka Pharmaceutical Co., Ltd.), or 200 µl of physiological saline alone was injected into the peritoneal cavities of the mice. The groups of mice treated as described above were named the ER-TR7 group and control group. The mice were reared for eight days while giving 3% DSS water. Then, the mice in each group were sacrificed, and their large intestines were collected to determine their lengths.

After length determination of the collected large intestines, some portions were excised and transferred into 1.5-ml tubes and frozen in liquid nitrogen. 1 ml of RNA-Bee (TEL-TEST, Inc.) per 50 mg of tissue was added. The tissue was homogenized, and 200 µl of chloroform (Sigma-Aldrich) was added to the resulting suspension. The mixture was gently mixed and then cooled on ice for about five minutes, and centrifuged in a centrifuge (Centrifuge 5417R, Eppendorf) at 12,000 rpm and 4°C for 15 minutes. After centrifugation, 500 µl of the supernatant was transferred to a fresh 1.5-ml tube, and an equal volume of isopropanol (500 µl; Sigma-Aldrich) was added thereto. The solution was mixed, and then 1 µl of glycogen (Invitrogen) was added thereto. The mixture was cooled on ice for 15 minutes, and then centrifuged at 12,000 rpm and 4°C for 15 minutes. Next, RNA precipitate obtained after washing three times with 1,000 µl of 75% ethanol (Sigma-Aldrich) was air-dried for 30 minutes to one hour, and then dissolved in Otsuka distilled water (Otsuka Pharmaceutical Co., Ltd.). The solution was 100 times diluted with Otsuka distilled water. The RNA concentrations of extracted samples in UV plates (Corning Costar) were determined using a plate reader (POWER Wave XS; BIO-TEK). The concentrations of the obtained RNA samples were adjusted to 500 ng/20 µl. The samples were heated at 68°C for three minutes in a BLOCK INCUBATOR (ASTEC), and cooled on ice for ten minutes. After cooling on ice, 80 µl of RT PreMix solution (composition: 18.64 µl of 25 mg MgCl₂ (Invitrogen), 20 µl of 5x Buffer (Invitrogen), 6.6 µl of 0.1 M DTT (Invitrogen), 10 µl of 10 mM dNTP mix (Invitrogen), 2 µl of Rnase Inhibitor (Invitrogen), 1.2 µl of MMLV Reverse Transcriptase (Invitrogen), 2 µl of Random primer (Invitrogen), and sterile distilled water (Otsuka distilled water; Otsuka Pharmaceutical Co., Inc.)), which had been prepared in advance, was added to the samples. The mixtures were heated in a BLOCK INCUBATOR at 42°C for one hour and at 99°C for five minutes, and then cooled on ice. cDNAs (100 µl) were prepared by the procedure described above.

PCR was carried out using the prepared cDNAs in the following composition. 1 µl of cDNA was mixed with 12.5 µl of SYBR Premix EX Taq (TAKARA), 11.3 µl of sterile distilled water (Otsuka distilled water), and 0.1 µl of primers (50 pmol/µl; Table 1). The resulting mixtures were reacted in Real-time PCR Dice (TAKARA) with 40 cycles of 95°C for 5 seconds and 60°C for 30 seconds. After reaction, the expression level of each fibrosis marker gene was determined relative to the expression level of β-actin gene as an internal standard.

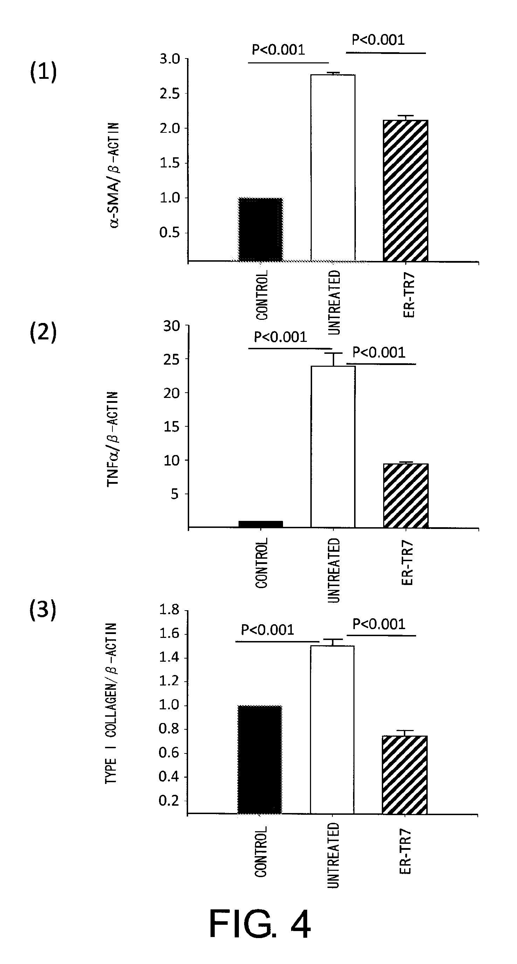

The result showed that the expression levels of all tested genes were reduced in the ER-TR7-treated group when compared to the control group. This suggests that ER-TR7 administration results in suppression of fibrosis (Fig. 4).

### [Example 5] ER-TR7 Immunocytochemistry of normal human pulmonary fibroblasts

Normal human pulmonary fibroblasts (1 x 10⁴ cells; CELL APPLICATION) were cultured for one day in HLF Growth Medium (CELL APPLICATION) containing 10% fetal calf serum (CELL APPLICATION) and 10 µg/ml lipopolysaccharide (*Escherichia Coli* 055:B5; Sigma-Aldrich) in 6-well plates (Corning Costar) with a gap cover glass (24 x 25 mm; Matsunami Glass Ind., Ltd.). The cells were washed three times with PBS, and then ice-cold methanol (Wako Pure Chemical Industries Ltd.) was added thereto. The cells were incubated at 4°C for 15 minutes. After washing three times with PBS, a BlockAce solution (Snow Brand Milk Products Co., Ltd.) was added, and then the cells were incubated at room temperature for two hours. Then, ER-TR7 (0.4 mg/ml) was added to the BlockAce solution at a ratio of 1:500, and the cells were incubated at room temperature for two hours. After washing three times with PBST (0.05% Tween20) for ten minutes, 1/2000 volume of Alexa Fluor (R) 488 Goat Anti-rat IgG (Molecular Probes) and 1/5000 volume of TO-PRO3 (Molecular Probes) were added to PBST. The mixture was added to the cells, and the cells were incubated at room temperature for two hours in the dark. After washing three times with PBST for ten minutes, the cells were mounted with Fluorescent Mounting Medium (DAKO Cytomation), and observed under a fluorescence microscope (Leica TCS SPE; Leica Microsystems).

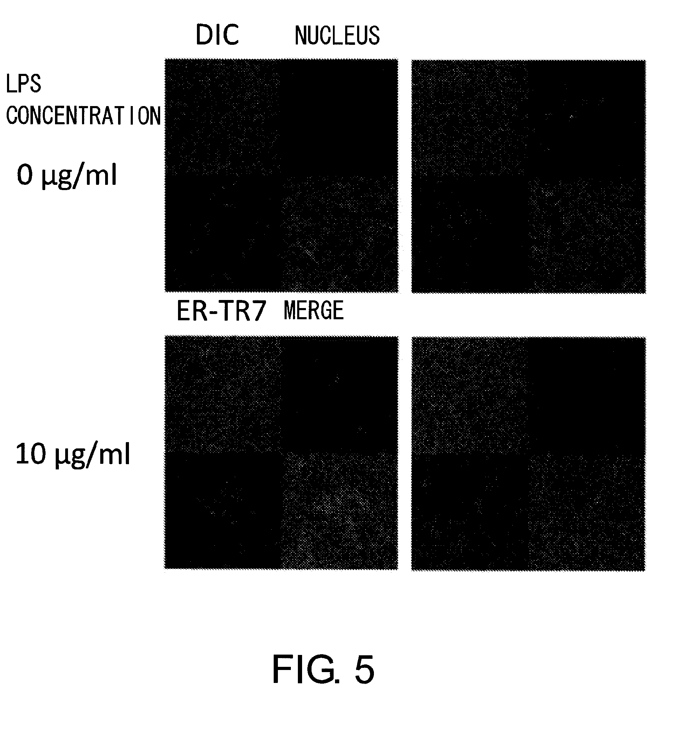

The result showed that normal human pulmonary fibroblasts expressed ER-TR7 antigens (Fig. 5).

### [Example 6] Identification of ER-TR7 antigen in normal human pulmonary fibroblast extract

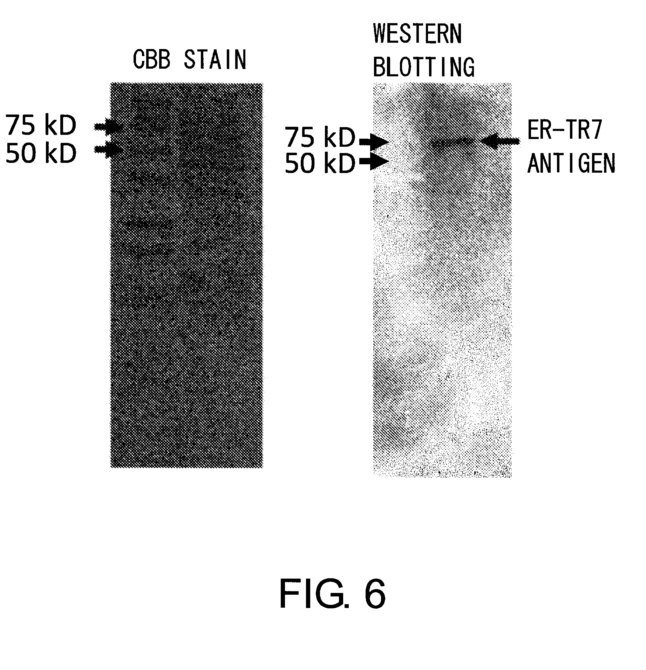

Normal human pulmonary fibroblasts were cultured until 80% confluent (approximately 5 x 10⁷ cells) in HLF Growth Medium containing 10% fetal calf serum in a T150 flask (Corning Costar). After washing three times with PBS, HLF Growth Medium containing 10% fetal calf serum and 10 µg/ml lipopolysaccharide (*Escherichia Coli* 055:B5) was added to the flask. The cells were cultured for three days. After washing three times with PBS, 500 µl of M-PER Mammalian Protein Extraction Reagent (PIERCE) was added to prepare cell extract. 8 µl of the obtained cell extract was mixed with 8 µl of 2x Sample Buffer (composition: 62.5 mM Tris-HCl (pH 6.8; Invitrogen), 25% glycerol (Wako Pure Chemical Industries Ltd.), 2% SDS (Nacalai Tesque), 350 mM DTT (Sigma Aldrich), 0.01% Bromophenol Blue (Sigma-Aldrich)), and this was heated in a BLOCK INCUBATOR (ASTEC) at 99°C for five minutes. After heating, the total volume was subjected to SDS-polyacrylamide gel electrophoresis (electrophoresis chamber: AE-6530M RAPIDAS mini slab gel electrophoresis chamber (ATTO); concentrating gel: 125 mM Tris-HCl (pH 6.8; Invitrogen), 0.01% SDS (Nacalai Tesque), 3.75% acrylamide solution (29:1 = acrylamide (Invitrogen) : bisacrylamide (Invitrogen)); resolving gel: 375 mM Tris-HCl (pH 8.8), 0.01% SDS, and 7.5% acrylamide solution; electrophoresis buffer: 25 mM Tris-HCl, 192 mM glycine (Wako Pure Chemical Industries Ltd.), and 0.1% SDS (pH 8.3)). Electrophoresis was carried out at 100 V for 40 minutes using Power Pac Basic (BIORAD). After electrophoresis, the samples were transferred onto Immobilon-P membrane (MILLIPORE) in a MinitransBlot Cell (BIORAD) at 100 V for 40 minutes. Then, the membrane was incubated in a BlockAce solution at room temperature for one hour. ER-TR7 (0.4 mg/ml) was mixed with a BlockAce solution at a ratio of 1:500, and the membrane was incubated in this solution at room temperature for two hours. After washing three times with TBST (50 mM Tris, 150 mM NaCl, and 0.01% Tween20) for five minutes, HRP GOAT anti-rat whole A (GE Healthcare Bioscience) was added to TBST at a ratio of 1:2000. The membrane was incubated in the solution at room temperature for one hour, and then washed three times with TBST for five minutes. Donkey Anti-Goat IgG (H+L) (Jackson ImmunoReseach Laboratories) was added to TBST at a ratio of 1:2000. The membrane was incubated in the solution at room temperature for one hour. After washing three times with TBST for five minutes, the membrane was soaked in SuperSignal West Dura Extended Duration Substrate (PIRCE). The signal was detected with FluorChem (AlphaInnotech).
As a result, a signal was detected at around 70 kDa. This suggests that this protein is the antigen of ER-TR7 and is deeply involved in the progression of fibrosis (Fig. 6).

### [Example 7] Confirmation of the presence of ER-TR7-positive cells in various tissues

### (1) Accumulation of ER-TR7-positive cells in lung tissues of pulmonary emphysema model mice

Porcine pancreatic esterase (PPE, four units; Calbiochem-Novabiochem) was intratracheally administered to C57BL/6J mice (female, five to six weeks old; CLEA Japan Inc.). The mice were fed for three weeks after administration, and then lung tissues were collected. Mice without PPE administration were used as a control group.

The collected lung tissue samples were embedded in OCT compound (Miles), an embedding medium for cryosectioning, and sliced into thin sections using a Cryostat (Carl Zeiss). The obtained sections were fixed with acetone (Wako Pure Chemical Industries Ltd.) for ten minutes, and then washed with phosphate buffer. ER-TR7 antibody (rat monoclonal antibody, 1 µg/ml; BMA) was added as the primary antibody, and the sections were incubated at room temperature for one hour. Next, after the secondary antibody reaction was carried out using peroxidase-labeled anti-rat IgG (200 times dilution), DAB substrate (Nichirei Bioscience) was added for color development. Then, nuclear staining was carried out using Lillie-Mayer hematoxylin (Muto Pure Chemicals Co.). The samples were observed under a light microscope (Leica Microsystems). The antibody binding was visualized as brown signals.

From the result described above, the presence of ER-TR7-positive cells in fibrotic lung tissues was confirmed. Specifically, it was demonstrated that fibrotic lung tissues could be a therapeutic target for the agents of the present invention.

### (2) Accumulation of ER-TR7-positive cells in pancreatic tissues of type 2 diabetes model mice

Gestational Day 14 C57BL/6JcL mice (CLEA Japan Inc.) were reared and allowed to deliver. 10 mg/ml Streptozotocin (SIGMA) was subcutaneously injected at 20 µl/head to day two postnatal female mice. The mice were fed with CE-2 diet (CLEA Japan Inc.) and sterile water until four weeks old. When the mice became 4 weeks old, they were fed with High Fat Diet (CLEA Japan Inc.) and sterile water for two weeks and pancreatic tissues were collected.

Immunostaining with ER-TR7 antibody was carried out by the same procedure described above. Then, the antibody binding was visualized as brown signals. From the result described above, the presence of ER-TR7-positive cells in fibrotic pancreatic tissues was confirmed. Specifically, it was demonstrated that fibrotic pancreatic tissues could be a therapeutic target for the agents of the present invention.

### (3) Accumulation of ER-TR7-positive cells in the brain of Parkinson's disease model mice

Gestational Day 14 of C57BL/6JcL mice (CLEA Japan Inc.) were reared and allowed to deliver. MPTP (Sigma-Aldrich Japan), which selectively destroys only dopamine neurons, was administered at 30 mg/kg into eight-week-old female mice for three consecutive days. The mice were reared, and one week after the initial administration their brain samples were excised.

The collected brain tissue samples were embedded in OCT compound (Miles), an embedding medium for cryosectioning, and sliced into thin sections using a Cryostat (Carl Zeiss). The obtained sections were fixed with phosphate buffer containing 4% PFA (Nacalai Tesque) for ten minutes, and then washed with deionized water. ER-TR7 (100 times dilution; BMA) was added as the primary antibody, and the sections were incubated at 4°C overnight. Next, Alexa488-labeled goat anti-rat IgG antibody (200 times dilution; Invitrogen) was added as the secondary antibody, and the sections were incubated at room temperature for 30 minutes. Then, the antibody binding was visualized.

From the result described above, the presence of ER-TR7-positive cells in fibrotic brain tissues was confirmed. Specifically, it was demonstrated that fibrotic brain tissues could be a therapeutic target for the agents of the present invention.

### (4) Accumulation of ER-TR7-positive cells in heart tissues of cardiomyopathy model mice

DOX (15 mg/kg; KYOWA HOKKO) was administered into the peritoneal cavities of C57BL/6J mice (male, eight weeks old; CLEA Japan Inc.). The mice were fed for one week after administration, and then heart tissues were collected.

Immunostaining with ER-TR7 was carried out by the same procedure described above. Then, the antibody binding was visualized as brown signals.

From the result described above, the presence of ER-TR7-positive cells in fibrotic cardiac tissues was confirmed. Specifically, it was demonstrated that fibrotic cardiac tissues could be a therapeutic target for the agents of the present invention.

### (5) Accumulation of ER-TR7-positive cells in the colonic tissues of ulcerative colitis model mice

The ulcerative colitis model mice were prepared by allowing C57BL/6J mice (female, six weeks old; CLEA Japan Inc.) to freely drink high-concentration chlorine water containing 3% dextran sulfate sodium (DSS; Wako Pure Chemical Industries Ltd.) for eight days. Then, large intestines were collected.

Immunostaining with ER-TR7 antibody was carried out by the same procedure described above. Then, the antibody binding was visualized as brown signals.

From the result described above, the presence of ER-TR7-positive cells in fibrotic colonic tissues was confirmed. Specifically, it was demonstrated that fibrotic colonic tissues could be a therapeutic target for the agents of the present invention.

### (6) Accumulation of ER-TR7-positive cells in kidney tissues of renal fibrosis model mice

The renal fibrosis model mice were prepared by performing unilateral ureteral obstruction (UUO) to C57BL/6JcL mice (female, eight weeks old; CLEA Japan Inc.). The renal fibrosis mouse model has excellent reproducibility, and is thus widely used as an experimental mouse model for renal fibrosis (American journal of pathology (2003) 163:4; 1261-1273). Mice were anesthetized with Ketalar/xylazine and underwent laparotomy. The ureters were exposed and the right ureter was ligated at two sites with 4-0 surgical suture. The peritoneum and skin were closed with 1-0 surgical suture. Eight days after, the kidney tissues were collected.

Immunostaining with ER-TR7 antibody was carried out by the same procedure described above. Then, the antibody binding was visualized as brown signals. From the result described above, the presence of ER-TR7-positive cells in fibrotic kidney tissues was confirmed. Specifically, it was demonstrated that fibrotic kidney tissues could be a therapeutic target for the agents of the present invention.

### (7) Accumulation of ER-TR7-positive cells in the liver of cirrhosis model mice

Carbon tetrachloride (10 µl; Sigma-Aldrich) was mixed with 90 µl of mineral oil (Sigma-Aldrich). The mixture was injected into the peritoneal cavities of C57BL/6J mice (female, five to six weeks old; CLEA Japan Inc.) twice a week for four weeks (seven times) to induce hepatic fibrosis. Then, carbon tetrachloride was additionally administered to the mice twice a week for two weeks (11 times in all) to induce cirrhosis. The mice were fed for two weeks after the above treatment, and then livers were collected.

Immunostaining with ER-TR7 antibody was carried out by the same procedure described above. Then, the antibody binding was visualized as brown signals.

From the result described above, the presence of ER-TR7-positive cells in fibrotic liver tissues was confirmed. Specifically, it was demonstrated that fibrotic liver tissues could be a therapeutic target for the agents of the present invention.

### Industrial Applicability

Fibrosis-suppressing agents of the present invention comprising ER-TR7 as an active ingredient have a therapeutic or preventive effect on diseases with excessive tissue fibrosis, by suppressing the fibrosis in body tissues. Since the fibrosis-suppressing agents of the present invention suppress the expression of α-SMA, TNFα, and type I collagen, which are fibrosis marker genes, in fibrotic tissues, they are extremely useful in suppressing tissue fibrosis. Methods for treating or preventing fibrotic diseases by administering the fibrosis-suppressing agents of the present invention can effectively improve fibrotic lesions through medical treatment, and could be excellent therapeutic methods that are useful for improving patients' QOL.

In addition, the present invention is not only medically applicable for humans but can also be expected to be used in the pet industry and animal husbandry, for example, to treat or prevent chronic fibrotic diseases of pets, or to prevent diseases of farm animals by administration, when antibodies against ER-TR7 antigen homolog of various animals are prepared.

## Claims

1. A fibrosis-suppressing agent comprising ER-TR7 or a functionally equivalent substance as an active ingredient.

2. The agent of claim 1, wherein the substance has an ER-TR7 antigen-targeting activity.

3. The agent of claim 1, wherein the substance has an activity of enhancing the degradation of an ER-TR7 antigen.

4. The agent of claim 1, wherein the substance has an activity of inhibiting the synthesis of an ER-TR7 antigen.

5. The agent of claim 1, wherein the substance has an activity of neutralizing the activity of an ER-TR7 antigen.

6. The agent of claim 1, wherein the substance has an activity of inhibiting the modification of an ER-TR7 antigen.

7. The agent of claim 1, wherein the substance has an activity of enhancing the demodification of an ER-TR7 antigen.

8. The agent of any one of claims 1 to 7, wherein the production or accumulation of an ER-TR7 antigen in each organ is inhibited.

9. The agent of any one of claims 1 to 8, which is used for treating or preventing a fibrotic disease.

10. A method for producing a fibrosis-suppressing agent comprising as an active ingredient an antibody, which comprises the step of preparing the antibody using an ER-TR7 antigen.

11. A method for treating or preventing a fibrotic disease, which comprises the step of administering the agent of any one of claims 1 to 9 to a subject.

12. Use of ER-TR7 or a functionally equivalent substance in the manufacture of a fibrosis-suppressing agent.
